# EUROPEAN PATENT APPLICATION

(11) **EP 4 428 222 A1**
(43) Date of publication of application: **11.09.2024**
(21) Application number: 23386021.2
(22) Date of filing: 10.03.2023
(51) Int. Cl.: C12M 1/42, C12M 3/06

(54) **DEVICES AND METHODS FOR ELECTROPORATION**

(71) Applicant: Cambridge Enterprise, Ltd., Cambridge Cambridgeshire CB2 1TN (GB); NATIONAL INSTITUTES OF HEALTH, Rockville, MD 20852-3804 (US)
(72) Inventor: The designation of the inventor has not yet been filed
(74) Representative: J A Kemp LLP

(57) **Abstract**

The present invention provides devices and methods for electroporation. Embodiments of the invention provide an electroporation device, the device having: a microfluidic channel with a fluid inlet and a fluid outlet; and a pair of interdigitated electrodes formed within said channel, wherein said electrodes are coated with PEDOT:PSS on a contact surface which is in contact with fluid in the channel. Other embodiments of the invention provide a method of performing electroporation on cells in a sample, the method including the steps of: introducing the sample into a chamber having a pair of electrodes each having a coating of PEDOT:PSS on a contact surface which is in contact with the sample; and applying oscillating stimulation voltage signals to the sample using the electrodes, wherein the stimulation voltage signals are applied so as to stimulate electroporation in the cells.

## Description

The present invention relates to devices and methods for electroporation. It is particularly, but not exclusively concerned with devices and method for electroporation which use conducting polymer electrodes.

Electroporation, or electropermeabilization, is a well-established technique in which cell membranes are transiently disrupted by pulsed electric fields to allow for the transport of otherwise impermeable molecules [1, 2]. While there are a variety of physical [3], chemical [4], and biological [5-8] methods to transfer biomolecules across the cell membrane, electroporation has remained a popular technique since the 1980's due to its ease of use, universal applicability, and reasonable efficiency [2, 9, 10]. Commercial electroporation devices apply pulsed electric fields with defined amplitude and duration in cuvettes or pipettes with embedded electrodes. These electrodes, often fabricated out of aluminum, stainless-steel, platinum, or graphite, are arranged as parallel plates to apply a uniform electric field across cells within the cuvette [11]. While widely used, these bulk electroporation systems require high voltage inputs and specialized capacitor discharge equipment to generate the required electric field intensity [12]. In addition to high input voltage, bulk electroporation traditionally suffers from electric field distortion, heat generation, local pH variation, and metal ion dissolution which negatively impacts both electroporation efficiency and cell viability [12].

To overcome these challenges, recent work has focused on miniaturization with microfluidic-based electroporators [13-15] as well as improving the electrode/electrolyte interface [16].

Microfluidic-based electroporation leverages the unique properties of micro-scale processing. Electrodes that are microns apart can generate required field intensities with low input voltage, provide uniform field distribution across individual cells, and rapidly dissipate heat all within a highly controlled fluidic and chemical environment [17, 18], thereby addressing many of the drawbacks of bulk electroporation. Furthermore, low volume microchannels enable the processing of individual cells to interrogate heterogeneity in cell populations, identify rare cells, and substantially reduce consumption of expensive reagents. These systems can be fabricated with additive manufacturing [19], conventional microfabrication techniques [20, 21], while the use of transparent materials such as polydimethylsiloxane (PDMS) and glass can further allow for real-time imaging to investigate electroporation processes at cellular resolution.

Interdigitated microelectrodes have long been used for a variety of cellular and molecular assays such as dielectrophoretic cell positioning, biomolecule concentration, and electroporation [15]. Their popularity stems from their ease of fabrication, electric field uniformity, and label free manipulation. These advantages reduce variation and allow for more consistent cell stimulation. A two-electrode interdigitated microelectrode system only requires a single metal layer thus reducing the number of alignment, photolithography and deposition steps and significantly reducing fabrication complexity. Other microelectroporation systems use more complex electrode arrays [28], optoelectronics [29], or microelectromechanical systems [30] to position and stimulate cells. While these systems offer greater single cell interrogation abilities, their added fabrication challenge does not make them as suitable to interrogate the electrode/electrolyte interface.

While miniaturization ameliorates many of the challenges with bulk electroporation, other issues such as local pH variation and metal ion dissolution are determined by choice of electrode material. At high voltages, metallic electrodes undergo electrochemical reactions at the electrode/electrolyte interface, inducing Faradaic currents. These irreversible Faradaic processes can produce gasses, pH changing species, chloride oxidation products, reactive oxygen species, and electrode dissolution [31-33]. Commonly used electrode materials such as aluminum and stainless steel, alongside inert metals such as platinum, and gold can all induce these electrochemical reactions changing the local electrolyte composition [34-36].

Conducting polymer electrodes have been shown to attenuate these irreversible electrochemical events. PEDOT:PSS (poly(3,4-ethylenedioxythiophene) doped with polystyrene sulfonate) is a conductive polymer that has been widely used in biomedical applications due to its biocompatibility, ease of processing, and reversible electrochemical properties [37-39]. PEDOT:PSS yields high capacitance electrodes due to its mixed electronic-ionic conductivity [40-42]. The high capacitance minimizes Faradaic processes and decouples the delivery of electric fields from surface electrochemistry. However, despite the apparent promise of PEDOT:PSS, previous attempts to use PEDOT:PSS electrodes for electroporation found that these electrodes reduce electroporation efficiency compared to uncoated metal electrodes [16].

The present invention aims to solve one or more of the above problems.

Aspects of the present invention aim to provide systems and methods which provide improved electroporation. In particular, aspects of the present invention aim to provide systems and methods which provide improve electroporation efficiency while maintaining cell viability.

Aspects of the present invention also aim to provide systems and methods which can perform successful electroporation over a wider range of operating conditions.

Aspects of the present invention provide systems and methods which use PEDOT:PSS coated electrodes. The inventors have surprisingly found that, contrary to previous published results, successful electroporation using PEDOT:PSS electrodes is possible and can show improved electroporation results compared to using uncoated metal electrodes in the same environment.

A first aspect of the present invention provides an electroporation device, the device having: a microfluidic channel with a fluid inlet and a fluid outlet; and a pair of interdigitated electrodes formed within said channel, wherein said electrodes are coated with PEDOT:PSS on a contact surface which is in contact with fluid in the channel.

The polymer coating reduces both impedance and electrochemical reactions at the electrode/electrolyte interface allowing for cells to be electrically stimulated with minimal changes to their chemical environment.

As demonstrated in the examples described below, the use of PEDOT:PSS electrodes can substantially improve electroporation efficiency while maintaining cell viability.

Preferably the microfluidic channel has a height perpendicular to a plane in which the electrodes are formed of no more than 500µm, preferably no more than 250µm, more preferably no more than 200µm, more preferably no more than 150µm. Preferably the microfluidic channel has a height perpendicular to the plane in which the electrodes are formed of at least 20µm, preferably at least 50µm.

Typical cell sizes are in the range of 8-20µm. Therefore, absent other approaches to position cells which are intended to be subject to electroporation in consistent positions relative to the electrodes, a channel which has a significant height relative to the size of the cells can mean that the cells can be in highly variable positions relative to the electrodes and even that multiple cells can end up in the same inter-electrode position, but at different vertical spacings from the electrodes and thus experience very different electrical fields. Using a microfluidic channel restricts the relative vertical positioning of cells relative to the electrodes.

Preferably the electrodes have a width of no more than 50µm, more preferably no more than 25µm and ideally around 20µm. Preferably the spacing between the interdigitated electrodes is no more than 50µm, more preferably no more than 25µm and ideally around 20µm.

Thinner electrodes and/or more closely spaced electrodes are believed to create a more uniform electrical field environment for cells which are introduced into the channel for electroporation. The spacing of the electrodes and/or the size of the electrodes above are of a similar dimension to the cells themselves, which means that, in typical environments, a single cell will end up located between the electrodes and will be located in a relatively consistent position with respect to the electrodes and thereby all cells which are in the channel and which are subject to fields from the electrodes will experience substantially similar or identical fields and thus a high degree of consistency in the electroporation of individual cells can be achieved which can thus make the electroporation process more efficient and/or predictable.

The device may further include, or may be connected to, a controller arranged to control the potential applied to the electrodes, wherein the controller is arrange to apply an oscillating potential to said electrodes to cause electroporation in a sample in the chamber.

The controller may arranged to apply an oscillating potential with a peak-to-peak voltage of between 0.1 and 10 V for the purposes of electroporation. Alternatively or additionally, the controller may be arranged to apply an oscillating potential at a frequency of between 100 Hz and 100kHz. The inventors have found that these ranges are most suitable for electroporation, particularly with a 20µm separation between electrodes (which results in a field strength of 50 V/cm to 5 kV/cm for the indicated voltage ranges). Further, the inventors have found that the electroporation results using PEDOT:PSS electrodes in these ranges can significantly exceed the results using uncoated metal electrodes.

In certain embodiments the controller is arranged to apply a first signal to the electrodes in order to position cells in a sample in the channel relative to the electrodes and to apply a second, different signal to the electrodes in order to cause electroporation in cells in the sample.

For example the controller may be arranged to apply a first signal to the electrodes in order to cause dielectrophoresis in cells in the sample. In certain embodiments the controller may be arranged to, when applying the first signal, alternate positive dielectrophoresis pulses and negative dielectrophoresis pulses to the electrodes to cause the cells in the sample to be positioned between the electrodes and close to the contact surface.

These embodiments use two step dielectrophoretic positioning to pattern cells between the PEDOT:PSS electrodes in a uniform electric field. This can allow the full separation of field delivery from electrochemistry in a controlled environment and enable a broader set of stimulation conditions.

The advantages of microfluidic-based electroporation may be enhanced when the electrodes are used for other fluidic processes such as cell sorting or hydrodynamic control [22, 23], or other electronic processes such as electrophoresis, electroosmosis, or dielectrophoresis (DEP) [24, 25].

DEP in particular can be used to significantly improve electroporation efficiency by precisely positioning cells between microelectrodes. This minimizes electric field distortion, as the electric field gradient is uniform between electrodes close to the surface. There is however greater field intensity at the edges of each electrode as well as sharp reduction in field away from the surface which can be leveraged for dielectric cell patterning. DEP forces arise from non-uniform electric field gradients between particles and media with different dielectric properties. The magnitude of the force is proportional to the intensity of the electric field gradient while the direction of the force can be tuned towards the higher electric field gradient (pDEP) or towards the lower electric field gradient (nDEP) as a function of frequency. Alternating pDEP and nDEP can thus be used to precisely pattern cells between electrodes and close to the surface in regions with high field uniformity. Cells randomly arranged between electrodes can also distort the local electric field and reduce the transmembrane potential in neighboring cells in what is known as the shadowing effect. This nonuniformity in induced transmembrane potentials results in heterogeneity across the cell population and reduces transfection efficiency. Precisely patterning cells between the electrodes to position them within a locally uniform electric field and minimize shadowing can thereby reduce heterogeneity and improve transfection efficiency.

The devices of this aspect may be further improved by leveraging sensing capabilities of PEDOT:PSS electrodes to maximize electroporation efficiency [43]. For example, the large surface area to volume ratio and low impedance of polymeric electrodes allows for high signal to noise measurements that would enable closed-loop feedback for single-cell individualized electroporation [28], with significantly lower costs due to the ease of thin film processing.

The cells may be pumped into the microfluidic device. Whilst examples use a syringe for pumping, the method may be performed as a continuous flow process, or as a repeated small batch process in order to provide a continuous throughput of electroporated cells. In such arrangements a pump may be used to cause the flow of the sample into the device and/or a pump used to extract the electroporated sample from the device.

The device of this aspect may include some, all or none of the above described optional and preferred features, in any combination.

A second aspect of the present invention provides a method of performing electroporation on cells in a sample, the method including the steps of introducing the sample into a chamber having a pair of electrodes each having a coating of PEDOT:PSS on a contact surface which is in contact with the sample; and applying oscillating stimulation voltage signals to the sample using the electrodes, wherein the stimulation voltage signals are applied so as to stimulate electroporation in the cells.

The polymer coating reduces both impedance and electrochemical reactions at the electrode/electrolyte interface allowing for cells to be electrically stimulated with minimal changes to their chemical environment.

Moreover, the inventors have surprisingly found that, contrary to previous published results, successful electroporation using PEDOT:PSS electrodes is possible and can show improved electroporation results compared to using uncoated metal electrodes in the same environment. In particular, as demonstrated in the examples described below, the use of PEDOT:PSS electrodes can substantially improve electroporation efficiency while maintaining cell viability.

The method may further include the step of adjusting or controlling the positioning of the cells so as to position the cells relative to the electrodes prior to stimulating the cells. This may include applying oscillating positioning voltage signals

In certain embodiments the positioning of the cells results from dielectrophoresis (DEP) and the positioning signals are applied to cause such dielectrophoresis.

In certain embodiments the positioning signals include applying alternate positive dielectrophoresis pulses and negative dielectrophoresis pulses to position the cells in the sample between the electrodes and close to the contact surface.

These embodiments use two step dielectrophoretic positioning to pattern cells between the PEDOT:PSS electrodes in a uniform electric field. This can allow the full separation of field delivery from electrochemistry in a controlled environment and enable a broader set of stimulation conditions.

The advantages of microfluidic-based electroporation may be enhanced when the electrodes are used for other fluidic processes such as cell sorting or hydrodynamic control [22, 23], or other electronic processes such as electrophoresis, electroosmosis, or dielectrophoresis (DEP) [24, 25].

DEP in particular can be used to significantly improve electroporation efficiency by precisely positioning cells between microelectrodes. This minimizes electric field distortion, as the electric field gradient is uniform between electrodes close to the surface. There is however greater field intensity at the edges of each electrode as well as sharp reduction in field away from the surface which can be leveraged for dielectric cell patterning. DEP forces arise from non-uniform electric field gradients between particles and media with different dielectric properties. The magnitude of the force is proportional to the intensity of the electric field gradient while the direction of the force can be tuned towards the higher electric field gradient (pDEP) or towards the lower electric field gradient (nDEP) as a function of frequency. Alternating pDEP and nDEP can thus be used to precisely pattern cells between electrodes and close to the surface in regions with high field uniformity. Cells randomly arranged between electrodes can also distort the local electric field and reduce the transmembrane potential in neighboring cells in what is known as the shadowing effect. This nonuniformity in induced transmembrane potentials results in heterogeneity across the cell population and reduces transfection efficiency. Precisely patterning cells between the electrodes to position them within a locally uniform electric field and minimize shadowing can thereby reduce heterogeneity and improve transfection efficiency.

The method of this aspect can be used with a device of the above described first aspect, including some, all or none of the optional and preferred features of that aspect, but need not be.

For example, the chamber may be a microfluidic channel. In particular embodiments the microfluidic channel has a height perpendicular to a plane in which the electrodes are formed of no more than 500µm, preferably no more than 250µm, more preferably no more than 200µm, more preferably no more than 150µm. Preferably the microfluidic channel has a height perpendicular to the plane in which the electrodes are formed of at least 20µm, preferably at least 50µm.

Typical cell sizes are in the range of 8-20µm. Therefore, absent other approaches to position cells which are intended to be subject to electroporation in consistent positions relative to the electrodes, a channel which has a significant height relative to the size of the cells can mean that the cells can be in highly variable positions relative to the electrodes and even that multiple cells can end up in the same inter-electrode position, but at different vertical spacings from the electrodes and thus experience very different electrical fields. Using a microfluidic channel restricts the relative vertical positioning of cells relative to the electrodes.

Preferably the electrodes have a width of no more than 50µm, more preferably no more than 25µm and ideally around 20µm. Preferably the spacing between the interdigitated electrodes is no more than 50µm, more preferably no more than 25µm and ideally around 20µm.

Thinner electrodes and/or more closely spaced electrodes are believed to create a more uniform electrical field environment for cells which are introduced into the channel for electroporation. The spacing of the electrodes and/or the size of the electrodes above are of a similar dimension to the cells themselves, which means that, in typical environments, a single cell will end up located between the electrodes and will be located in a relatively consistent position with respect to the electrodes and thereby all cells which are in the channel and which are subject to fields from the electrodes will experience substantially similar or identical fields and thus a high degree of consistency in the electroporation of individual cells can be achieved which can thus make the electroporation process more efficient and/or predictable.

The signals used to stimulate the cells may have a peak-to-peak voltage of between 0.1 and 10 V. Alternatively or additionally, the signals used to stimulate the cells may have a frequency of between 100 Hz and 100kHz. The inventors have found that these ranges are most suitable for electroporation, particularly with a 20µm separation between electrodes (which results in a field strength of 50 V/cm to 5 kV/cm for the indicated voltage ranges). Further, the inventors have found that the electroporation results using PEDOT:PSS electrodes in these ranges can significantly exceed the results using uncoated metal electrodes.

The methods of this aspect may be further improved by leveraging sensing capabilities of PEDOT:PSS electrodes to maximize electroporation efficiency [43]. For example, the large surface area to volume ratio and low impedance of polymeric electrodes allows for high signal to noise measurements that would enable closed-loop feedback for single-cell individualized electroporation [28], with significantly lower costs due to the ease of thin film processing.

The methods of this aspect may include further steps of pre-processing and/or post-processing before and/or after the electroporation.

In particular, the cells may be pumped into the microfluidic device. Whilst examples use a syringe for pumping, the method may be performed as a continuous flow process, or as a repeated small batch process in order to provide a continuous throughput of electroporated cells. In such arrangements a pump may be used to cause the flow of the sample into the device and/or a pump used to extract the electroporated sample from the device.

The method of the above aspect may include some, all or none of the above-described optional and preferred features in any combination.

Unless indicated otherwise, any of the features (including the optional or preferred features) described in relation to one of the above aspects are equally applicable in combination with the devices, systems and methods of any of the other above-described aspects.

Embodiments of the invention are described below, by way of example, with reference to the accompanying figures in which:
Figure 1 shows an overview of devices according to the present invention and the testing environment used to test those devices;
Figure 2 shows the stimulation sequences used in embodiments of the present invention and in testing the devices of the embodiments described;
Figure 3 shows the time development of permeabilization and cell viability in tests of devices and methods according to embodiments of the present invention; and
Figure 4 shows the performance of devices and methods according to embodiments of the present invention under various operating conditions.

### Electrodes

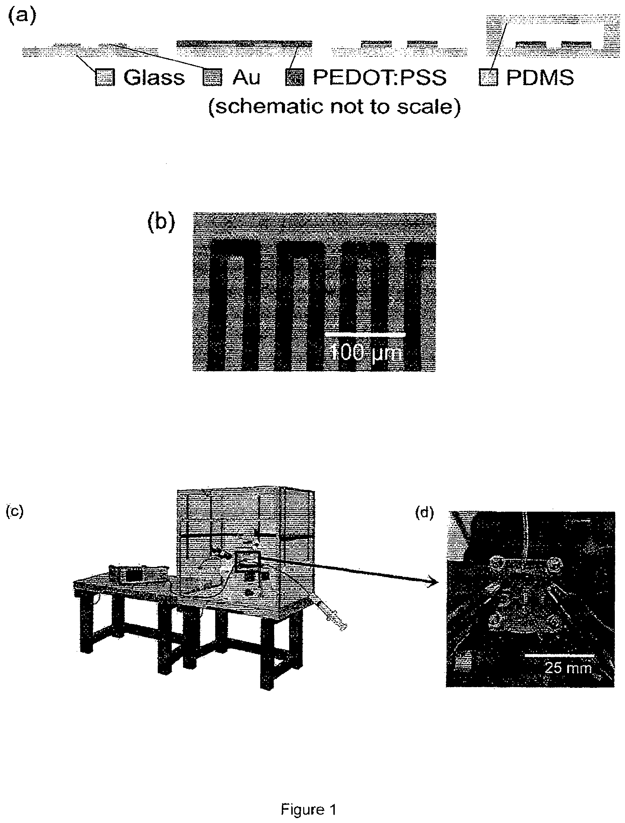

Figure 1(a) shows, schematically and not to scale, a cross sectional view of the steps in the manufacture of devices according to embodiments of the present invention.

Interdigitated microelectrodes with 20µm width and spacing were patterned onto glass substrates using standard microfabrication techniques such as those disclosed in [43]. Photomasks (JD PhotoData, Hitchin, United Kingdom) were first designed using AutoCAD (AutoDesk, San Rafael, CA). Glass substrates were prepared by washing in isopropanol (IPA), acetone, and de-ionized water (DI H₂O) and dehydrated on a hotplate at 150° C for 5 minutes. Then, a photoresist (AZ nLOF2035, Microchemicals, Ulm, Germany) was spin coated on the substrate at 500 rpm for 5 seconds, 3000 rpm for 45 seconds, and baked at 110° C for 60 seconds. After this, the substrate was loaded under the photomask in a mask aligner, exposed to 105 mJ/cm² i-line UV, and post exposure baked for 3 mins at 110° C. The substrate was then developed in AZ726 MIF developer for 29 seconds until the microelectrode pattern was visible in the resist. The surface was then activated with oxygen plasma for 60s and then 5nm of titanium and 100nm of gold are deposited using E-beam evaporation (Kurt J Lesker Company, Jefferson Hills, PA). The photoresist was removed by a lift-off process in acetone for 15 mins or until there was no longer ohmic contact measured between the electrodes.

The electrodes were coated with PEDOT:PSS. A PEDOT:PSS solution was prepared with a surfactant to improve film homogeneity and a cross-linker. This solution was spin-coated on the electrodes at 500 rpm for 5 seconds and 3000 rpm for 30 seconds and baked for 60 seconds at 110° C. This spin-coating was repeated a total of 3 times to produce a relatively thick (~575 nm) film before hard baking for 1 hour at 110° C. The hard-baked devices were then soaked in DI H₂O for 24 hours before lithographically patterning positive photoresist (AZ5214E, Microchemicals, Ulm, Germany) above the digits of the electrodes. Exposed PEDOT:PSS was removed using a reactive ion etch (Oxford Instruments, Abingdon, United Kingdom) and the remaining photoresist was removed using acetone leaving the final PEDOT:PSS microelectrodes.

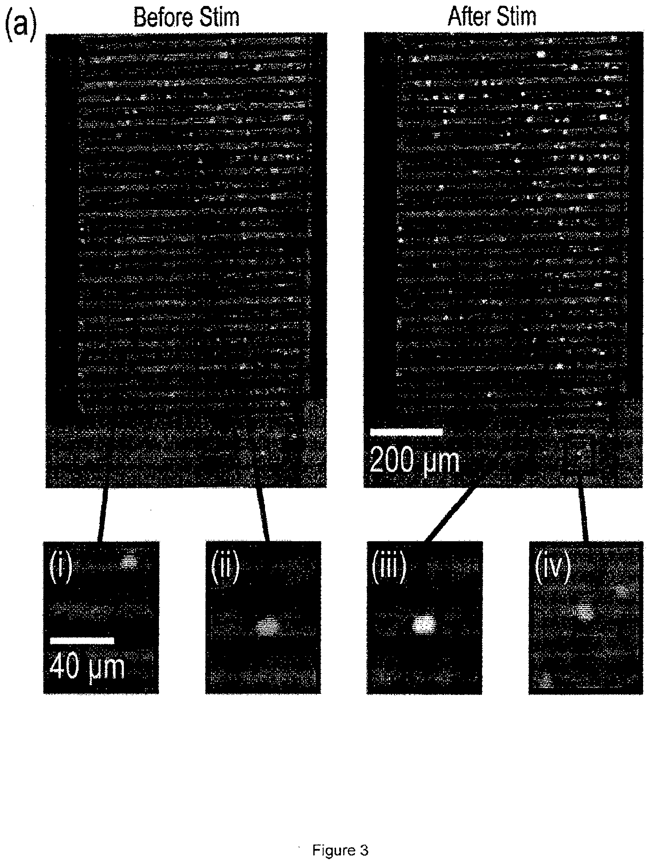

Figure 1(b) shows a section of the final electrode array. Figure 3(a) shows the complete interdigitated pattern of electrodes in a device according to an embodiment of the present invention.

### Microfluidics

A 150 µm height, 1mm width microfluidic channel was fabricated using conventional soft lithography [44]. First, a thick negative photoresist (SU-8 2100, Kayaku Advanced Materials, Westborough, MA) was spin-coated on a silicon substrate at 500 rpm for 10 seconds and 2000 rpm for 30 seconds. The edge bead was removed using propylene glycol monomethyl ether acetate (PGMEA, Sigma-Aldrich, St. Louis, MO) and the film was baked at 65° C for 5 mins, then 95° C for 20 mins. Then, the film was aligned to a photomask, exposed with 240 mJ/cm² UV, and baked at 65° C for 5 mins and 95° C for 10 mins. Finally, the film was developed in PGMEA with agitation for 10 mins, rinsed with fresh PGMEA, followed by a second rinse with IPA and air-dried with nitrogen.

This mold was then placed in a vacuum chamber with 100 µL Sigmacote (Sigma-Aldrich, St. Louis, MO) as a mold-release agent. PDMS (Sylgard 184, Dow Coming, Midland, MI) was prepared at a 10:1 base to cross-linker ratio, poured onto the mold, and baked at 80° C for 1 hour. The cured PDMS was peeled off the mold, diced, and had inlet and outlet holes punched. Both the microelectrodes and microfluidics were O₂ plasma surface activated, the channel was aligned over the microelectrodes under a microscope, and the surfaces bonded.

The final cross-sectional configuration of the electrodes and the microfluidic channel is shown schematically in the right-hand image ofFigure 1(a).

### Packaging

Electrical connection pins (Ossila, Sheffield, United Kingdom) were clamped to contact pads at the edge of the glass substrate and epoxied in place to ensure a strong contact. A 3D printed holder was then screwed over the entire assembly for added robustness. Needles and tubing were attached to the inlet and outlet holes to establish fluidic contact and a syringe was used to push media and cells through the channel.

Figure 1(d) shows the exterior of a finished device with fluidic connectors entering/exiting top and bottom, and electrical connections being made on the left and right sides.

### Cell Culture

U87 cells were cultured in Dulbecco's Modified Eagle Medium (DMEM, Gibco/Invitrogen) supplemented with 10% fetal bovine serum (FBS, HyClone), 100 units/ml penicillin, and 100 µg/ml streptomycin at 37° C and 5% CO₂. Media was changed every 3-4 days with a 1:5 cell-split ratio and cells were harvested with 0.25% trypsin (Life Technologies). For electroporation experiments, cells were spun down at 400 × g for 7 mins and re-suspended in an electroporation buffer with a density of 1×10⁶ cells/mI.

### Electroporation Buffer

A known buffer with 0.01×PBS, a conductivity of 0.03 S/m, and supplemented with sucrose to an osmolarity of 180 mOsm/L was used [25]. One part RNAse free 1× PBS (Invitrogen, Carlsbad, CA) was added to 99 parts of DI water. Then 60.58 g/L of D(+) sucrose was added to adjust the osmolarity of the buffer to 180 mOsm/L.

### Electroporation Experiments

The device was placed in a confocal microscope to visualize cells during stimulation as seen in Figure 1(c). Initially two fluorescent dyes, calcein-acetoxymethyl (calcein-AM, final concentration 5 µg/ml) and propidium iodide (PI, final concentration 5 µg/ml) were added to harvested cells to verify electroporation. In subsequent experiments, either PI was added to cells before to visualize membrane permeability, or Calcein AM was added to visualize cell viability. The devices were sterilized by flowing through 1 ml of 70% ethanol in DI H₂O, and then rinsed with 1ml of DI H₂O.

All electroporation experiments were done within 20 minutes of harvesting cells. In experiments to determine membrane permeability, PI dye was added to cells immediately prior to stimulation and a 3ml syringe was used to flow cells through the device.

In experiments to determine cell viability, cells were stimulated on the device, flushed out, plated in a 6 well plate, and cultured in an incubator for 48 hours. Cells were stained with Calcein AM 90 mins before imaging.

The voltage was controlled by a 30 MHz arbitrary waveform generator (SDG 1000X; RS Pro, Corby, UK) with sequential stimulation as follows:
(1) pDEP - AC signal 1 V peak-to-peak (Vpp), 100 kHz, duration 5 seconds
(2) nDEP - AC signal 1Vpp, 10 kHz, duration 5 seconds
(3) transfection - bipolar square wave with amplitude ranging from 0.1-10 Vpp and pulse width ranging from 100-100,000 Hz, duration 400ms

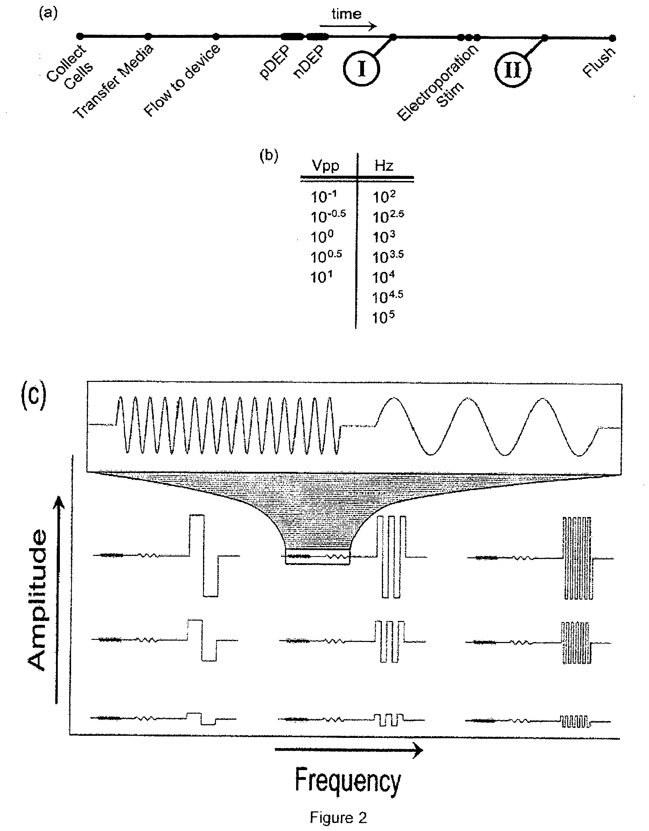

Figure 2 illustrates the cell stimulation sequence (Figure 2(a)) and the various test signals (Figures 2(b)-(c)) that were used to study and determine optimal stimulation conditions.

Figure 2(a) is a timeline of the experiment wherein cells are transferred into electroporation buffer and injected into the device. pDEP is then used to bring cells close to the surface and nDEP is used to pattern cells between electrodes before the electroporation stimulation. Permeabilized cells are flushed out of the chip. Images of a device at time points (I) and (II) are shown in Figure 3(a).

Figure 2(b) is a tabulated set of test conditions showing the peak-to-peak amplitudes of the electroporation signal (Vpp) and the frequencies used. Figure 2(c) shows, schematically, some of the signal waveforms used in the testing, in which the preliminary pDEP and nDEP waveforms (magnified at the top of the Figure) are unchanged between each test, whilst the amplitude and frequency of the subsequent electroporation pulses are varied.

Five image fields were taken for each test condition and images were processed with CellProfiler [45] to determine percentage of cells that expressed a fluorescent signal and the data was plotted.

### Results and Discussion:

Electroporation was initially characterized by stimulating cells on the device under a fluorescent microscope after staining with Calcein AM and PI. Calcein AM is a membrane permeable dye that is not fluorescent until the acetoxymethyl ester group is cleaved by intracellular esterases in living cells and converted into Calcein. Calcein is then green fluorescent (ex 494 nm, em 5 17nm) and is a marker for cell viability. PI on the other hand is impermeable to intact cell membranes. Once inside a cell, PI will intercalate nucleic acids and produce a strong red fluorescence (ex 535 nm, em 617nm), thereby serving as a marker for membrane permeability.

Figure 3(a) is an overlay of brightfield, green fluorescence, and red fluorescence images of cells inside a microfluidic channel patterned between electrodes. Living cells with undisrupted membranes are green due to the hydrolysis of calcein AM, dead cells are red due to intercellular PI and lack of esterase activity. Living cells with disrupted membranes appear yellow due to the overlay of the green and red fluorescence signals. In the greyscale images, the red cells in the left image (I) have been circled (magnified image (i)). The same cells show red in the right image (II). In the left image, the non-circled cells are all green (magnified image (ii)), whilst in the right image, all non-circled cells within the interdigitated electrode pattern are yellow (magnified image (iii)), whilst those outside the electrodes are green (magnified image (iv)).

After DEP patterning but before the electroporation stimulation, >95% of visible cells were alive (Figure 3(a)-I - green cells). After stimulation with a 1 Vpp, 1 kHz bipolar square wave, all living cells that are between electrodes express both the calcein and PI signal (yellow cells), while living cells that are not between electrodes only express the calcein signal (green cells - Figure 3(a)-II). This demonstrates that cells between electrodes are sufficiently disrupted during the electroporation stimulation to allow for the transport of PI across their membranes.

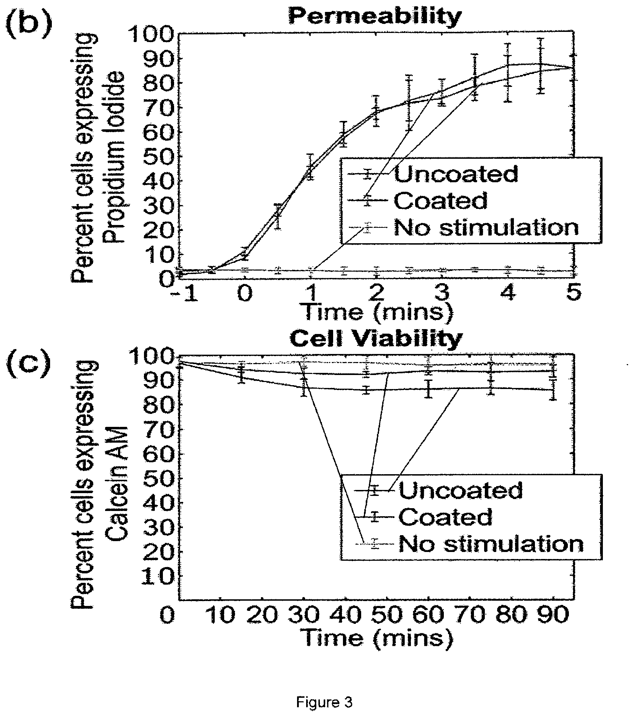

Figure 3(b) shows the time course of the PI signal within cells stimulated with both uncoated Au and coated PEDOT:PSS electrodes. PI diffuses into cells soon after stimulation and a majority of cells express the dye within 4 minutes using either electrode type.

To assess cell viability, the same stimulation conditions were repeated without PI, as the dye can be toxic to cells. Cells were imaged on the chip at 15 minute intervals for 90 minutes after stimulation as seen in Figure 3(c). It can be seen that there is a significant difference in cell viability between PEDOT:PSS and Au electrodes, with the former electrodes having an average of 10% greater cell viability 90 mins after stimulation.

On-chip test conditions do not necessarily accurately represent the final use case as electroporation buffer is not an ideal culture medium and there is likely to have been variation in temperature and CO₂ pressure in the microscope chamber. To minimize these effects, further viability experiments with various stimulation conditions were carried out by flushing cells out of the device, changing the buffer to culture media, and culturing them under optimal conditions in an incubator at 37C with 5% CO₂ before measuring cell viability with the calcein assay. This protocol more closely resembles the conventional use of electroporation devices.

A matrix of potential operating conditions with variations in the amplitudes and frequencies tabulated in Figure 2(b) were tested. These voltages create local potential differences ranging from 50 to 5,000 V/cm in the 20 µm spacing between electrodes, reflecting values typically used in electroporation. Frequencies below 100 Hz were not included in the analysis due to visible production of large gas bubbles on Au electrodes that greatly disrupted cells in the channel. These experiments were done on three uncoated-electrode devices and three PEDOT:PSS coated-electrode devices.

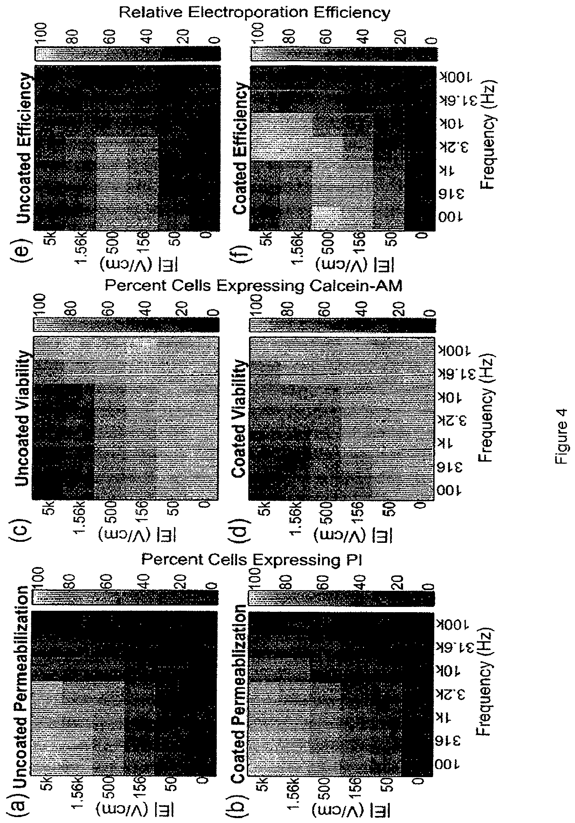

Figures 4(a) (Au electrodes - "uncoated") and 4(b) (PEDOT:PSS electrodes - "coated") illustrate the proportion of cells between electrodes that express PI 4 minutes after electroporation at the various operating conditions. Low frequency and high amplitude stimulation tends to cause a greater proportion of cells to uptake PI. With 5kV/cm field and 100 Hz frequency results in a PI signal within 100% of cells on both Au and PEDOT:PSS electrodes across all devices. This is consistent with previous understanding [20, 25] that membrane disruption is a function of both the transmembrane potential generated in an external electric field, as well as the duration of that stimulation. Larger amplitudes generate greater transmembrane potential while lower frequency results in longer duration of that potential, and ultimately more membrane disruption.

The inverse also holds true, high frequency and low amplitude stimulation results in very few cells up-taking PI. For example, 50 V/cm and 100kHz stimulation results in approximately 6% of cells expressing a PI signal while an average of 4% of unstimulated cells express a PI signal with 3% standard deviation between devices, a difference which is not significant.

It can be seen from Figures 4(a) and (b) that Au and PEDOT:PSS electrodes have very similar permeabilization efficiencies with field strengths greater than 500 V/cm and frequencies lower than 3.2 kHz. However, at lower field strengths and higher frequencies, PEDOT:PSS electrodes exhibit on average 8% higher electroporation efficiency than Au electrodes with 4% standard deviation between devices meaning that this is a significant difference.

Cell viability on the other hand tends to be low at high amplitudes and low frequencies. Figures 4(c) and 4(d) show the proportion of cells with a Calcein-AM signal 90 minutes after stimulation. A 5 kV/cm field at 100 Hz amplitude results in approximately 9% cell viability on Au electrodes and 17% viability on PEDOT:PSS electrodes with 6% standard deviation. There remains a significant difference in viability between Au and PEDOT:PSS electrodes at field strengths above 500 V/cm and frequencies below 10 kHz with PEDOT:PSS electrodes exhibiting up to 11% higher cell viability. At the other extreme, a 50 V/cm, 100 kHz field results in 95% and 96% viability on the Au and PEDOT:PSS electrodes respectively, an insignificant difference from the average 96% viability of unstimulated cells. This field intensity and frequency dependence of cell viability shares a mechanism with permeabilization efficiency wherein higher transmembrane potentials and durations result in greater membrane disruption and correspondingly greater cell death.

Optimizing electroporation efficiency entails maximizing permeabilization while minimizing cell death. Therefore, as a simplified metric of electroporation efficiency, pairwise multiplication of the permeabilization efficiency with the cell viability at different stimulation conditions was carried out, with the results shown in Figures 4(e) for Au electrodes and 4(f) for PEDOT:PSS electrodes.

As expected, electroporation efficiency is low in regions of low cell viability, such as when field intensities are high and frequencies low, as well as in regions where permeabilization is low such as when field intensities are low and frequencies high. Optimal electroporation occurs in the penumbral region where both permeabilization and viability are high, and it is these conditions in which PEDOT:PSS electrodes have two major advantages.

Firstly, PEDOT:PSS electrodes have both higher permeabilization and higher viability across all operating conditions, with up to 19% greater performance on the chosen electroporation metric in conventional operating conditions (500 V/cm, 100 Hz) and up to 63% improvement in other operating conditions (5k V/cm, 10 kHz).

In addition to the bulk improvement in efficiency, another major advantage is the broader range of operating conditions. A substantially higher proportion of the overall condition matrix for PEDOT:PSS shows efficiency readings of >80% (compare Fig. 4f to 4e). As optimal electroporation conditions are contingent on cell type [46], this improved electrode performance over a much broader range of stimulation parameters demonstrate that PEDOT:PSS electrodes have the potential for use across a wide range of cell types.

The foregoing description is exemplary in nature only, and the skilled person will understand that changes and variations on the disclosed embodiments are possible within the scope of the claims. The claims define the invention.

### REFERENCES

1. Hapala I. Breaking the barrier: methods for reversible permeabilization of cellular membranes. Crit Rev Biotechnol. 1997;17(2):105-122. doi:10.3109/07388559709146609
2. Neumann E, Schaefer-Ridder M, Wang Y, Hofschneider PH. Gene transfer into mouse lyoma cells by electroporation in high electric fields. EMBO J. 1982;1(7):841-845. doi:10.1002/j.1460-2075.1982.tb01257.x
3. Diacumakos EG. Methods for micromanipulation of human somatic cells in culture. Methods Cell Biol. 1973;7:287-311. doi:10.1016/s0091-679x(08)61783-5
4. Graham FL, van der Eb AJ. A new technique for the assay of infectivity of human adenovirus 5 DNA. Virology. 1973;52(2):456-467. doi:10.1016/0042-6822(73)90341-3
5. Hamer DH, Leder P. Expression of the chromosomal mouse βmaj-globin gene cloned in SV40. Nature. 1979;281(5726):35-40. doi:10.1038/281035a0
6. Mulligan RC, Howard BH, Berg P. Synthesis of rabbit β-globin in cultured monkey kidney cells following infection with a SV40 β-globin recombinant genome. Nature. 1979;277(5692):108-114. doi:10.1038/277108a0
7. Immordino ML, Dosio F, Cartel L. Stealth liposomes: review of the basic science, rationale, and clinical applications, existing and potential. Int J Nanomedicine. 2006;1(3):297-315.
8. Fraley R, Subramani S, Berg P, Papahadjopoulos D. Introduction of liposome-encapsulated SV40 DNA into cells. J Biol Chem. 1980;255(21):10431-10435.
9. Weaver JC, Chizmadzhev YuA. Theory of electroporation: A review. Bioelectrochem Bioenerg. 1996;41(2):135-160. doi:10.1016/S0302-4598(96)05062-3
10. Shi J, Ma Y, Zhu J, et al. A Review on Electroporation-Based Intracellular Delivery. Molecules. 2018;23(11):3044. doi:10.3390/molecules23113044
11. Potter H. Electroporation in biology: methods, applications, and instrumentation. Anal Biochem. 1988;174(2):361-373. doi:10.1016/0003-2697(88)90035-8
12. Canatella PJ, Karr JF, Petros JA, Prausnitz MR. Quantitative study of electroporation-mediated molecular uptake and cell viability. Biophys J. 2001;80(2):755-764.
13. Lee WG, Demirci U, Khademhosseini A. Microscale electroporation: challenges and perspectives for clinical applications. Integr Biol Quant Biosci Nano Macro. 2009;1(3):242-251. doi;10.1039/b819201d
14. Movahed S, Li D. Microfluidics cell electroporation. Microfluid Nanofluidics. 2010;10(4):703.
15. Geng T, Lu C. Microfluidic electroporation for cellular analysis and delivery. Lab Chip. 2013;13(19):3803-3821. doi:10.1039/C3LC50566A
16. Dijk G, Ruigrok HJ, O'Connor RP. PEDOT:PSS-Coated Stimulation Electrodes Attenuate irreversible Electrochemical Events and Reduce Cell Electropermeabilization. Adv Mater Interfaces. 2021;8(19):2100214. doi: 10.1002/admi.202100214
17. Wang S, Lee LJ. Micro-/nanofluidics based cell electroporation. Biomicrofluidics. 2013;7(1):011301. doi:10.1063/1.4774071
18. Bhattacharjee N, Horowitz LF, Folch A. Continuous-flow multi-pulse electroporation at low DC voltages by microfluidic flipping of the voltage space topology. Appl Phys Lett. 2016;109(16):163702. doi:10.1063/1.4963316
19. Waheed S, M. Cabot J, P. MacdonaldN, et al. 3D printed microfluidic devices: enablers and barriers. Lab Chip. 2016;16(11):1993-2013. doi:10.1039/C6LC00284F
20. Adamo A, Arione A, Sharei A, Jensen KF. Flow-through comb electroporation device for delivery of macromolecules. Anal Chem. 2013;85(3):1637-1641. doi:10.1021/ac302887a
21. Huang H, Wei Z, Huang Y, et al. An efficient and high-throughput electroporation microchip applicable for siRNA delivery. Lab Chip. 2010;11(1):163-172. doi;10.1039/C0LC00195C
22. Zhu T, Luo C, Huang J, Xiong C, Ouyang Q, Fang J. Electroporation based on hydrodynamic focusing of microfluidics with low dc voltage. Biomed Microdevices. 2010;12(1):35-40. doi:10.1007/s10544-009-9355-z
23. Wang J, Zhan Y, Ugaz VM, Lu C. Vortex-assisted DNA delivery. Lab Chip. 2010;10(16):2057-2061. doi:10.1039/C004472E
24. Wei Z, Li X, Zhao D, et al. Flow-Through Cell Electroporation Microchip Integrating Dielectrophoretic Viable Cell Sorting. Anal Chem. 2014;86(20):10215-10222. doi: 1 0.1 02 J /ac502294e
25. Jayasooriya V, Ringwelski B, Dorsam G, Nawarathna D. mRNA-based CAR T-cells Manufactured by Miniaturized Two-step Electroporation Produce Selective Cytotoxicity Toward Target Cancer Cells. Lab Chip. 2021;21(19):3748-3761. doi:10.1039/d1lc00219h
26. Pavlin M, Pavselj N, Miklavcic D. Dependence of induced transmembrane potential on cell density, arrangement, and cell position inside a cell system. IEEE Trans Biomed Eng. 2002;49(6):605-612. doi:10.1109/TBME.2002.1001975
27. Susil R, Šemfov D, Miklavčič D. Electric Field-Induced Transmembrane Potential Depends on Cell Density and Organizatio. *Electro- Magnetobiology.* 1998;17(3):391-399. doi:10.3109/15368379809030739
28. Zhang Z, Zheng T, Zhu R. Single-cell individualized electroporation with real-time impedance monitoring using a microelectrode array chip. Microsyst Nanoeng. 2020;6(1):1-10. doi:10.1038/s41378-020-00196-0
29. Georg Breunig H, Uchugonova A, Batista A, König K. Software-aided automatic laser optoporation and transfection of cells. Sci Rep. 2015;5(1):11185. doi:10.1038/srep1 1185
30. Fus-Kujawa A, Prus P, Bajdak-Rusinek K, et al. An Overview of Methods and Tools for Transfection of Eukaryotic Cells in vitro. Front Bioeng Biotechnol. 2021;9. Accessed November 16, 2022. https://www.frontiersin.org/articles/10.3389/fbioe.2021.701031
31. Turjanski P, Olaiz N, Maglietti F, et al. The Role of pH Fronts in Reversible Electroporation. PLOS ONE. 2011;6(4):e17303. doi:10.1371/journal.pone.0017303
32. Li Y, Wu M, Zhao D, et al. Electroporation on microchips: the harmful effects of pH changes and scaling down. Sci Rep. 2015;5(1):17817. doi:10.1038/srep17817
33. McHardy J, Robblee LS, Marston JM, Brummer SB. Electrical stimulation with Pt electrodes. IV. Factors influencing Pt dissolution in inorganic saline. Biomaterials. 1980;1(3):129-134. doi:10.1016/0142-9612(80)90034-4
34. Saulis G, Lape R, Pranevicite R, Mickevicius D. Changes of the solution pH due to exposure by high-voltage electric pulses. Bioelectrochemistry Amst Neth. 2005;67(1):101-108. doi:10.1016/j.bioelechem.2005.03.001
35. Kumsa DW, Hudak EM, Bhadra N, Mortimer JT. Electron transfer processes occurring on platinum neural stimulating electrodes: pulsing experiments for cathodic-first, charge-imbalanced, biphasic pulses for 0.566 ≤ k ≤ 2.3 in rat subcutaneous tissues. J Neural Eng. 2019;16(2):026018. doi:10.1088/1741-2552/aaf931
36. Vižintin A, Vidmar J, Ščančar J, Miklavčič D. Effect of interphase and interpulse delay in high-frequency irreversible electroporation pulses on cell survival, membrane permeabilization and electrode material release. *Bioelectrochemistry Amst Neth.* 2020;134:107523. doi:10.1016/j.bioelechem.2020.107523
37. Dijk G, Rutz AL, Malliaras GG. Stability of PEDOT:PSS-Coated Gold Electrodes in Cell Culture Conditions. Adv Mater Technol. 2020;5(3):1900662. doi:10.1002/admt.201900662
38. Donahue MJ, Sanchez-Sanchez A, Inal S, et al. Tailoring PEDOT properties for applications in bioelectronics. Mater Sci Eng R Rep. 2020;140:100546. doi:10.1016/j.mser.2020.100546
39. Inal S, Rivnay J, Suiu AO, Malliaras GG, McCulloch I. Conjugated Polymers in Bioelectronics. Acc Chem Res. 2018;51(6): 1368-1376. doi:10.1021/acs.accounts.7b00624
40. Berggren M, Malliaras GG. How conducting polymer electrodes operate. Science. 2019;364(6437):233-234. doi-10.1126/science.aaw9295
41. Stavrinidou E, Leleux P, Rajaona H, et al. Direct measurement of ion mobility in a conducting polymer. Adv Mater Deerfield Beach Fla. 2013;25(32):4488-4493. doi:10.1002/adma.201301240
42. Rivnay J, Leleux P, Ferro M, et al. High-performance transistors for bioelectronics through tuning of channel thickness. Sci Adv. 2015;1(4):e140025I. doi:10.1126/sciadv.1400251
43. Middya S, Curto VF, Fernández-Villegas A, et al. Microelectrode Arrays for Simultaneous Electrophysiology and Advanced Optical Microscopy. Adv Sci. 2021;8(13):2004434. doi:10.1002/advs.202004434
44. McDonald JC, Duffy DC, Anderson JR, et al. Fabrication of microfluidic systems in poly(dimethylsiloxane). Electrophoresis. 2000;21(l):27-40. doi:10.1002/(SICI)1522-2683(20000101)21:1<27::AID-ELPS27>3.0.CO;2-C
45. Stirling DR, Swain-Bowden MJ, Lucas AM, Carpenter AE, Cimini BA, Goodman A. CellProfiler 4: improvements in speed, utility and usability. BMC Bioinformatics. 2021 ;22(1):433. doi:10.1186/s 12859-021-04344-9
46. Jordan ET, Collins M, Terefe J, Ugozzoli L, Rubio T. Optimizing Electroporation Conditions in Primary and Other Difficult-to-Transfect Cells. J Biomol Tech JBT. 2008;19(5):328-334.

All of the above references are hereby incorporated by reference in their entirety.

## Claims

1. An electroporation device, the device having:
a microfluidic channel with a fluid inlet and a fluid outlet; and
a pair of interdigitated electrodes formed within said channel,
wherein said electrodes are coated with PEDOT:PSS on a contact surface which is in contact with fluid in the channel.

2. The electroporation device of claim 1 wherein the electrodes have a width of no more than 50µm, preferably no more than 20µm.

3. The electroporation device of claim 1 or claim 2 wherein the spacing between the interdigitated electrodes is no more than 50µm, preferably no more than 20µm.

4. The electroporation device of any one of the preceding claims wherein the microfluidic channel has a height perpendicular to a plane in which the electrodes are formed of no more than 250µm, preferably no more than 200µm, preferably no more than 150µm.

5. The electroporation device of any one of the preceding claims, further having a controller arranged to control the potential applied to the electrodes, wherein the controller is arrange to apply an oscillating potential to said electrodes to cause electroporation in a sample in the chamber.

6. The electroporation device of claim 5 wherein the control ler is arranged to apply a first signal to the electrodes in order to cause dielectrophoresis in cells in a sample in the chamber and to apply a second, different, signal to the electrodes in order to cause electroporation in cells in the sample.

7. The electroporation device of claim 6 wherein the controller is arranged to, when applying the first signal, alternate positive dielectrophoresis pulses and negative dielectrophoresis pulses to the electrodes to cause the cells in the sample to be positioned between the electrodes and close to the contact surface.

8. A method of performing electroporation on cells in a sample, the method including the steps of:
introducing the sample into a chamber having a pair of electrodes each having a coating ofPEDOT:PSS on a contact surface which is in contact with the sample; and
applying oscillating stimulation voltage signals to the sample using the electrodes, wherein the stimulation voltage signals are applied so as to stimulate electroporation in the cells.

9. The method according to claim 8 wherein the electrodes have a width of no more than 50µm, preferably no more than 20µm.

10. The method according to any one of claims 8 to 9 wherein the spacing between the interdigitated electrodes is no more than 50µm, preferably no more than 20µm.

11. The method according to any one of claims 8 to 11 further including the step of applying oscillating positioning voltage signals so as to position the cells relative to the electrodes prior to stimulating the cells.

12. The method according to claim 11 wherein the positioning of the cells results from dielectrophoresis (DEP) and the positioning signals are applied to cause such dielectrophoresis.

13. The method according to claim 12 wherein applying the positioning signals includes applying alternate positive dielectrophoresis pulses and negative dielectrophoresis pulses to position the cells in the sample between the electrodes and close to the contact surface.

14. The method according to any one of claims 8 to 13 wherein the chamber is a microfluidic channel.

15. The method according to claim 14 wherein the microfluidic channel has a height perpendicular to a plane in which the electrodes are formed of no more than 250µm, preferably no more than 200µm, preferably no more than 150µm.
